# EUROPEAN PATENT SPECIFICATION

(11) **EP 2 328 563 B1**
(45) Date of publication and mention of the grant of the patent: **20.03.2013**
(21) Application number: 09741481.7
(22) Date of filing: 24.08.2009
(51) Int. Cl.: A61K 9/20, A61K 9/16, A61K 9/26, A61K 9/28, A61K 9/32, A61K 31/405, A61K 31/397

(54) **SOLUBILITY ENHANCING PHARMACEUTICAL FORMULATION**
PHARMAZEUTISCHE FORMULIERUNG ZUR VERBESSERUNG DER LÖSLICHKEIT
PREPARATION PHARMACEUTIQUE AMELIORANT LA SOLUBILITE

(30) Priority: 22.08.2008 TR 200806300
(43) Date of publication of application: 08.06.2011
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2009/000108
(87) International publication number: WO 2010/021608

(56) References cited:
- EP-A- 1 741 427
- WO-A-02/058696
- WO-A-2005/011638
- WO-A-2006/134604
- WO-A-2008/101723

## Description

### Field of the invention

The present invention relates to the pharmaceutical formulations comprising a therapeutically active substance which has a solubility problem in combination with other therapeutically active substances, and the methods for the preparation thereof, and the use thereof.

### Background of the invention

The present invention provides a combination effective in reducing elevated total cholesterol (total-C), low-density lipoprotein cholesterol (LDL-C), apolipoprotein B (Apo B), and triglycerides (TO), and in increasing high-density lipoprotein cholesterol (HDL-C) in patients with mixed hyperlipidemia or primary hypercholesterolemia (heterozygous familial and non-familial hypercholesterolemia); in reducing elevated total-C and LDL-C in patients with homozygous familial hypercholesterolemia (HoFH); and in reducing elevated sitosterol and campesterol levels in patients with homozygous familial sitosterolemia. This effect which is provided by the combination according to the present invention is hereinafter referred as "the desirable effect". The mentioned combination comprises ezetimibe as a cholesterol absorption inhibitor and atorvastatin as an HMG-CoA reductase inhibitor.

Ezetimibe is a cholesterol absorption inhibitor with a chemical name of (3*R*,4*S*) -1- (4-fluorophenyl) -3- [(3*S*)-3-(4-fluorophenyl)-3-hydroxypropyl] -4- (4-hydroxyphenyl) -2-azetidinone (Formula I).

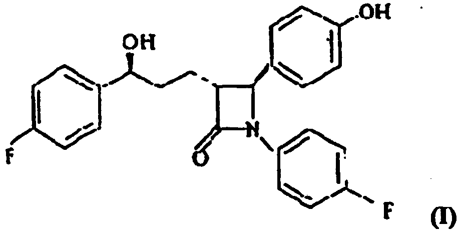

Ezetimibe is disclosed for the first time in the patent numbered USS631365 A (USRE37721E, US5767115 A, US5846966 A, W09508532 A1 and EP0720599 B1 are in the same patent family). Processes for preparing ezetimibe, pharmaceutical compositions comprising ezetimibe and the use of ezetimibe as a hypocholesterolemic agent are also disclosed in the same prior art. Also disclosed is that the use of ezetimibe in combination with HMG-CoA reductase inhibitors such as lovastatin, pravastatin, fluvastatin, simvastatin and atorvastatin is effective in reducing the plasma cholesterol levels and in the treatment of atherosclerosis.

Ezetimibe is an anti-hyperlipidemic medication suitable for oral use. It lowers serum cholesterol concentration by selectively inhibiting the absorption of cholesterol and phytosterols structurally similar to cholesterol in the intestine. Its mechanism of action is complementary to HMG-CoA reductase inhibitors. So, when ezetimibe and HMG-CoA reductase inhibitors are co-administered, the cholesterol lowering effect increases synergistically.

Atorvastatin is an HMG-CoA reductase inhibitor with a chemical name of (β*R*, δ*R*)-2-(4-Fluoro-phenyl)- β,δ -dihydroxy-5-(1-methylethyl)-3-phenyl-4-[(phenylamino)carbonyl]-1H-pyrrole-1-heptanoic acid (Formula II).

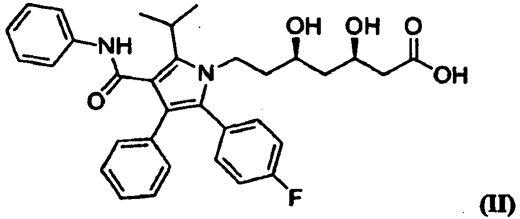

Atorvastatin is disclosed for the first time in the patent numbered EP409281 B1 (EP1061073 B1 and US5273995 A are in the same patent family). Processes for preparing atorvastatin and the use of atorvastatin as a cholesterol biosynthesis inhibitor are also disclosed in the same prior art.

Atorvastatin is a hypolipidemic drug which is a selective competitive inhibitor of 3-hydroxy-3-methyl-glutaryl-coenzyme A (HMG-CoA) reductase. Atorvastatin is the only drug in this class that can reduce both elevated LDL cholesterol and triglycerides in patients with hypercholesterolemia.

Synergistically increasing cholesterol lowering effect of ezetimibe and HMG-CoA reductase inhibitors was proven by the various clinical trials:
- Davis HR, Pula KK, Alton KB, Burrier RE & Watkins RW. The Synergistic Hypocholesterolemic Activity of the Potent Cholesterol Absorption Inhibitor, Ezetimibe, in Combination With 3-Hydroxy-3-Methylglutaryl Coenzyme A Reductase Inhibitors in Dogs. Metabolism 2001; 50(10):1234-1241
- Sudhop T, Von Bergmann K. Cholesterol absorption inhibitors for the treatment of hypercholesterolaemia. Drugs 2002; 62(16):2333-47
- Gagné C, MD; Gaudet D, MD PhD; Bruckert E, MD PhD. Efficacy and Safety of Ezetimibe Coadministered With Atorvastatin or Simvastatin in Patients With Homozygous Familial Hypercholesterolemia. Circulation 2002; 105; 2469-2475
- Davidson M.H., Ballantyne C.M., Kerzner B., Melani L., Sager P.T., Lipka L., Strony J., Suresh R., Veltri E., For Ezetimibe Study Group. Efficacy and safety of ezetimibe coadministered with statins: randomised, placebo-controlled, blinded experience in 2382 patients with primary hypercholesterolemia. Journal of Clinical Practice, August 2004, 58(8): 746-755
- Gagne C.; Bays H.E.; Weiss S.R.; Mata P.; Quinto K.; Melino M.; Cho M.; Musliner T.A.; Gumbiner B.1; Ezetimibe Study Group. Efficacy and safety of ezetimibe added to ongoing statin therapy for treatment of patients with primary hypercholesterolemia. Am J Cardiol 2002; 90: 1084-1091
- Christie M. Ballantyne, John Houri, Alberto Notarbartolo, Lorenzo Melani, Leslie J. Lipka, Ramachandran Suresh, Steven Sun, Alexandre P. LeBeaut, Philip T. Sager ve Enrico P. Veltri. Effect of ezetimibe coadministered with atorvastatin in 628 patients with primary hypercholesterolemia: a prospective, randomized, double-blind trial. Circulation 2003; 107; 2490-2415
- Lipka L.J.. Ezetimibe: a first-in-class, novel cholesterol absorption inhibitor. Cardiovascular Drug Reviews, 21(4); 293-312
- Kosoglu T, Meyer I, Veltri EP, et al. Pharmacodynamic interaction between the new selective cholesterol absorption inhibitor ezetimibe and simvastatin. Br J Clin Pharmacol. 2002

The present invention is directed to obtain a combination of ezetimibe as an effective hypocholesterolemic agent and atorvastatin as a potent hypolipidemic agent, which provides the desirable effect, depending on synergistically increasing cholesterol lowering effect of ezetimibe and HMG-CoA reductase inhibitors.

The object of the invention is to provide a dosage form such as tablet by combining pharmaceutically acceptable, non-toxic and therapeutically effective amount of ezetimibe and atorvastatin in a manner so as to obtain the desirable effect.

When the solid dosage forms, such as tablets, are taken orally, in many cases, the drug must first dissolve in aqueous gastrointestinal fluids before exhibiting its effect. But, because many drugs such as ezetimibe are small organic molecules with low solubility, dissolution problems arise. And having low dissolution rates limit their bioavailability.

One of the known techniques applied to address the solubility problem of poorly soluble drugs is particle size reduction. Because the dissolution rate of a particulate solid depends on the surface area and the surface area increases as the particle size reduces, reducing particle size may increase dissolution rate.

However, particle size reduction is not always effective at increasing the dissolution rate of a drug. Because, many hydrophobic drugs have a strong tendency to agglomerate while being transformed into larger particles during the dosage form manufacturing process depending on an overall decrease in effective surface area.

Another technique applied to increase the surface area is nanoparticulate technology. But, there are some barriers faced during the processes for obtaining nanoparticles such as technical and mechanical limitation of breaking the drug particles into the size of nano particles and the stabilization of these small drug particles in the dosage form.

So, there is a need for novel methods for overcoming the solubility problem of ezetimibe to be able to combine ezetimibe and the other therapeutical agents such as HMG-CoA reductase inhibitors on which ezetimibe has a synergistic effect.

The compounds comprising a first pharmacological moiety covalently linked to a second pharmacological moiety through a physiologically labile linkage are disclosed in the patent application numbered W02006110882 A2. First pharmacological moiety is selected from HMG-CoA reductase inhibitors and second pharmacological moiety is selected from the group of medicines including cholesterol absorption inhibitors such as ezetimibe. HMG-CoA reductase inhibitors are defined to be atorvastatin, pravastatin, simvastatin, lovastatin, fluvastatin, cerivastatin and rosuvastatin. The mechanism of action of ezetimibe and HMG-CoA reductase inhibitors is complementary according to the patent application. It is also stated in the application that this synergistic effect had been proven by clinical trials. As a result, this invention relates to novel compounds consisting of two pharmacological moieties and the use thereof. The solubility problem of ezetimibe is not mentioned in the application, accordingly there is not any solution for this problem presented in the application.

Stable antihyperlipoproteinemic oral pharmaceutical formulations which comprise ezetimibe, an HMG-CoA reductase inhibitor, disintegrants and glidants are disclosed in the patent application numbered WO2006134604 A1. HMG-CoA reductase inhibitors are defined to be atorvastatin, simvastatin and rosuvastatin. This invention discloses examples of formulations with known excipients. But, the solubility problem of ezetimibe is not mentioned in the application, accordingly there is not any solution for this problem presented in the application.

A method for treating or preventing sitosterolemia comprising administering at least one sterol absorption inhibitor (ezetimibe) optionally in combination with at least one lipid lowering agent is disclosed in the patent application numbered WO02058696 A2. Lipid lowering agent is defined to be an HMG-CoA reductase inhibitor selected from atorvastatin, pravastatin, simvastatin, lovastatin, fluvastatin, pitavastatin and rosuvastatin. This invention particularly relates to the medical use of ezetimibe. The solubility problem of ezetimibe is not mentioned in the application, accordingly there is not any solution for this problem presented in the application.

The above-mentioned patent applications are directed to combine ezetimibe and HMG-CoA reductase inhibitors because of their synergistic effect. But, the solubility problem that does not allow such combinations to work efficiently is not mentioned in the patents/patent applications. So, there is still a need for various solutions which allow formulators to combine ezetimibe and HMG-CoA reductase inhibitors so as to achieve the desirable effect.

### Summary of the invention

The present invention relates to a process for the preparation of a pharmaceutical composition comprising therapeutically effective amount of ezetimibe or a pharmaceutically acceptable salt thereof and therapeutically effective amount of atorvastatin or a pharmaceutically acceptable salt thereof for use in the manufacture of a medicament for the treatment of primary hypercholesterolemia (heterozygous familial and non-familial hypercholesterolemia), mixed hyperlipidemia, homozygous familial hypercholesterolemia and homozygous familial sitosterolemia, characterized in that ezetimibe or a pharmaceutically acceptable salt thereof and atorvastatin or a pharmaceutically acceptable salt thereof are preformulated in a series of manufacturing process steps.

According to the present invention, the manufacturing process which provides a formulation so as to obtain the desirable effect is as follows:
- atorvastatin or a pharmaceutically acceptable salt thereof, a pharmaceutically acceptable diluent and optionally other pharmaceutically acceptable excipients are mixed and afterwards sieved to obtain the first preformulate;
- ezetimibe or a pharmaceutically acceptable salt thereof is dissolved by the addition thereof into a granulation solution comprising a pharmaceutically acceptable surfactant which the surfactant is dissolved in a solvent comprising a cellulose derivative and/or a suitable pure solvent or a solvent mixture;
- a pharmaceutically acceptable diluent is granulated with a granulation solution;
- the granules obtained in the previous step are dried and sieved, and optionally mixed with other pharmaceutically acceptable excipients to obtain the second preformulate;
- both of the preformulates are optionally mixed to obtain a homogenous tablet, and the final mixture is optionally mixed with the other pharmaceutically acceptable excipients and made ready for tablet press;
- both of the preformulates are optionally fed separately to the tablet press machine to obtain a stratified tablet;
- tablets obtained in the previous step are optionally film-coated.

### Detailed description of the invention

The present invention is directed to obtain a combination of ezetimibe and atorvastatin, which provides the desirable effect, depending on synergistically increasing cholesterol lowering effect of ezetimibe and HMG-CoA reductase inhibitors. But, as defined before, there is a solubility problem which is faced during the studies.

To make sure the combination of ezetimibe and atorvastatin provides the desirable effect, on the one hand solubility problem of ezetimibe must be overcome, and on the other hand therapeutically effective amounts of the active components and the suitable composition of the excipients must be found.

Surprisingly, it is found that a pharmaceutical composition comprising ezetimibe (or a pharmaceutically acceptable salt thereof), atorvastatin (or a pharmaceutically acceptable salt thereof) and a pharmaceutically acceptable surfactant, wherein ezetimibe (or a pharmaceutically acceptable salt thereof) and atorvastatin (or a pharmaceutically acceptable salt thereof) are preformulated, has an optimum efficacy in the treatment of the various cardiovascular diseases.

According to another aspect of the invention, a pharmaceutical composition comprising ezetimibe (or a pharmaceutically acceptable salt thereof) in a specific amount, atorvastatin (or a pharmaceutically acceptable salt thereof) in a specific amount, a pharmaceutically acceptable surfactant in an adequate amount, at least one pharmaceutically acceptable diluent in an adequate amount and optionally one or more pharmaceutically acceptable excipients selected from the additives such as binders, disintegrants, glidants and lubricants, has an optimum efficacy in the treatment of the various cardiovascular diseases.

Solubility problem is solved by applying a series of manufacturing process steps to ezetimibe and atorvastatin due to their specific properties.
- In the first step, atorvastatin or a pharmaceutically acceptable salt thereof, a pharmaceutically acceptable diluent and optionally other pharmaceutically acceptable excipients are mixed together and the first preformulate is obtained.
- In the second step, granulation solution is obtained by dissolving a pharmaceutically acceptable surfactant in a solvent containing a cellulose derivative and/or a suitable pure solvent or a solvent mixture. Ezetimibe or a pharmaceutically acceptable salt thereof is dissolved by the addition thereof into a granulation solution comprising a pharmaceutically acceptable surfactant used for its solubility enhancing effect. The granules obtained by spraying the granulation solution comprising ezetimibe onto a pharmaceutically acceptable diluent and afterwards dried and sieved exhibit more than 90% dissolution in the first 10 minutes in the dissolution medium of ezetimibe, while ezetimibe normally tends to agglomerate. The second preformulate is obtained by optionally mixing the granules with the other pharmaceutically acceptable excipients.
- Then, the final mixture obtained by mixing both of the preformulates are optionally mixed with the other pharmaceutically acceptable excipients and made ready for tablet press; or both of the preformulates are fed separately to the tablet press machine to **obtain a stratified tablet.**
- Finally, tablets obtained in the previous step are optionally film-coated.

The term "the various cardiovascular diseases" as used herein refers to primary hypercholesterolemia (heterozygous familial and non-familial hypercholesterolemia), mixed hyperlipidemia, homozygous familial hypercholesterolemia and homozygous familial sitosterolemia.

The terms "in a specific amount", "in an adequate amount" and "optionally" as used herein refer to ezetimibe (or a pharmaceutically acceptable salt thereof) in an amount of from about 0.1 to 20% by weight, atorvastatin (or a pharmaceutically acceptable salt thereof) in an amount of from about 1 to 40% by weight, a pharmaceutically acceptable surfactant in an amount of from about 0.01 to 5% by weight, at least one pharmaceutically acceptable diluent in an amount of more than about 60% by weight and one or more pharmaceutically acceptable excipients selected from the additives such as binders, disintegrants, glidants and lubricants which are used when needed, which all of them are preferred to obtain the desirable effect.

"Pharmaceutically acceptable salt of atorvastatin" may be derived from inorganic bases selected from alkali metals such as sodium, lithium and potassium, alkaline earth metals such as calcium and magnesium, and aluminium, zinc, ammonium, and the like; or organic bases selected from arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, isopropylamine, lysine, methylglucosamine, morpholine, piperazine, piperidine, polyamine resins, procaine, purine, theobromine, triethylamine, trimethylamine, basic ion-exchange resins such as tripropylamine, cyclic amines, substituted amines, primary, secondary, tertiary amines, and the like; and is preferably calcium salt.

Pharmaceutically acceptable surfactants may be selected from polyoxyethylene-sorbitan-fatty acid esters (polysorbates), sodium lauryl sulfate, polyoxyethylene alkyl ethers, sorbitan fatty acid esters, polyethylene glycols, polyoxyethylene castor oil derivatives, docusate sodium, quaternary ammonium compounds, sugar esters of fatty acids, glycerides of fatty acids, and the like. According to the invention, pharmaceutical composition comprises preferably sodium lauryl sulfate as a surfactant

Pharmaceutically acceptable diluents may be selected from lactose, microcrystalline cellulose, starch, pregelatinized starch, modified starch, calcium phosphate (dibasic and/or tribasic), calcium sulfate trihydrate, calcium sulfate dihydrate, calcium carbonate, kaolin, lactilol, powder cellulose, dextrose, dextrates, dextrin, sucrose, maltose, fructose, mannitol, sorbitol, xylitol, and the like. The pharmaceutical composition of the invention comprises preferably lactose as diluent. Lactose may be selected from the different forms of lactose such as lactose DC, lactose monohydrate, lactose anhydrous, and the like. Besides the lactose one more diluent selected from the group listed above can also be added to the composition when needed.

Pharmaceutically acceptable binders may be selected from starches (such as potato starch, corn starch, wheat starch), sugars such as sucrose, glucose, dextrose, lactose and maltodextrin, natural and synthetic gums (such as acacia), gelatin, cellulose derivatives (such as microcrystalline cellulose, HPC, HEC, HPMC, carboxymethylcellulose, methylcellulose, ethylcellulose), polyvinylpyrrolidone, polyethylene glycol, waxes, calcium carbonate, calcium phosphate, alcohols (such as sorbitol, xylitol, mannitol), water, and the like. Binder is present in an amount within the range of preferably 0 to 10% by weight, more preferably 0.1 to 5% by weight.

Pharmaceutically acceptable disintegrants may be selected from starch (such as potato starch, corn starch), sodium starch glycolate, pregelatinized starch, cellulose derivatives (such as croscarmellose sodium, microcrystalline cellulose), polyvinylpyrrolidone, crospovidone, alginic acid, sodium alginate, clays (such as xanthan gum or Veegum), ion-exchange resins, effervescent systems such as those utilizing food acids and alkaline carbonate components, and the like. Preferably croscarmellose sodium is used. Disintegrant is present in an amount within the range of preferably 0 to 10% by weight, more preferably 1 to 5% by weight.

Pharmaceutically acceptable glidants may be selected from silicon dioxide, magnesium trisilicate, powder cellulose, starch, talk, tribasic calcium phosphate, metallic stearates, calcium silicate, metallic lauryl sulfates, and the like. Preferably silicon dioxide is used. Glidant is present in an amount up to 1% by weight.

Pharmaceutically acceptable lubricants may be selected from metallic stearates (such as magnesium stearate, calcium stearate, aluminium stearate), fatty acid esters (such as sodium stearyl fumarate), fatty acids (such as stearic acid), fatty alcohols, glyceryl behenate, mineral oils, paraffins, hydrogenated vegetable oils, leucine, polyethylene glycols, metallic lauryl sulfates (such as sodium lauryl sulfate, magnesium lauryl sulfate), sodium chloride, sodium benzoate, sodium acetate, talk, and the like. Preferably magnesium stearate is used. Lubricant is present in an amount within the range of preferably 0 to 10% by weight, more preferably 0.25 to 5% by weight.

In addition, antioxidants, protecting agents, stabilizing agents, solubility enhancers, electrolytes, sweeteners, colorants, coating agents, and the like may be used in the formulation.

The examples according to the invention are given below. These examples are given to explain the invention, but does not limit the scope of the invention.

### Examples

### Example 1.

| **Ingredients** | **Quantity (mg)** |
|---|---|
| Atorvastatin Calcium * | 10.84 |
| Ezetimibe | 10 |
| Lactose | 210.46 |
| Croscarmellose sodium | 8 |
| Calcium carbonate | 5.5 |
| Silicon dioxide | 0.2 |
| Magnesium stearate | 2 |
| Sodium lauryl sulfate | 3 |
| Core tablet weight | 250 |
| Coating material | 8 |

| | |
|---|---|
| * Equivalent to 10 mg of atorvastatin | |

### Example 2.

| **Ingredients** | **Quantity (mg)** |
|---|---|
| Atorvastatin Calcium * | 21.68 |
| Ezetimibe | 10 |
| Lactose | 348.9 |
| Croscarmellose sodium | 13 |
| Magnesium stearate | 2.92 |
| Sodium lauryl sulfate | 3.5 |
| Core tablet weight | 400 |
| Coating material | 16 |

| | |
|---|---|
| * Equivalent to 20 mg of atorvastatin | |

### Example 3.

| **Ingredients** | **Quantity (mg)** |
|---|---|
| Atorvastatin Calcium * | 43.36 |
| Ezetimibe | 10 |
| Lactose | 587.26 |
| Croscarmellose sodium | 28 |
| Calcium carbonate | 22.5 |
| Magnesium stearate | 4.88 |
| Sodium lauryl sulfate | 4 |
| Core tablet weight | 700 |
| Coating material | 24 |

| | |
|---|---|
| * Equivalent to 40 mg of atorvastatin | |

### Example 4.

| **Ingredients** | **Quantity (mg)** |
|---|---|
| Atorvastatin Calcium * | 86.72 |
| Ezetimibe | 10 |
| Lactose | 1145.18 |
| Croscarmellose sodium | 45 |
| Silicon dioxide | 2.1 |
| Sodium lauryl sulfate | 11 |
| Core tablet weight | 1300 |
| Coating material | 30 |

| | |
|---|---|
| * Equivalent to 80 mg of atorvastatin | |

## Claims

1. A process for the preparation of a pharmaceutical composition comprising therapeutically effective amount of ezetimibe or a pharmaceutically acceptable salt thereof and therapeutically effective amount of atorvastatin or a pharmaceutically acceptable salt thereof, **characterized in that** ezetimibe or a pharmaceutically acceptable salt thereof and atorvastatin or a pharmaceutically acceptable salt thereof are preformulated in a series of manufacturing process steps comprising,
a) preparing the preformulate of atorvastatin by mixing and afterwards sieving atorvastatin or a pharmaceutically acceptable salt thereof, a pharmaceutically acceptable diluent and optionally other pharmaceutically acceptable excipients together;
b) preparing the preformulate of ezetimibe by dissolving ezetimibe or a pharmaceutically acceptable salt thereof in a solution comprising a pharmaceutically acceptable surfactant wherein the surfactant is in a solvent comprising a cellulose derivative and/or a suitable pure solvent or a solvent mixture and thus obtaining a granulation solution and then granulating a pharmaceutically acceptable diluent with said granulation solution, followed by drying and sieving the granules;
c) mixing the preformulate of atorvastatin prepared in step a), the preformulate of ezetimibe prepared in step b) and optionally other pharmaceutically acceptable excipients.

2. The process according to claim 1, **characterized in that** both of the preformulates are mixed to obtain a homogenous tablet, the final mixture is optionally mixed with the other pharmaceutically acceptable excipients and made ready for tablet press; or both of the preformulates are fed separately to the tablet press machine to obtain a stratified tablet.

3. A pharmaceutical composition prepared according to claim 1 or claim 2.

4. The pharmaceutical composition prepared according to claim 3, **characterized in that** the pharmaceutical composition comprises, based on the weight of the core tablet,
- ezetimibe or a pharmaceutically acceptable salt thereof in an amount of from about 0.1 to 20% by weight,
- atorvastatin or a pharmaceutically acceptable salt thereof in an amount of from about 1 to 40% by weight,
- a pharmaceutically acceptable surfactant in an amount of from about 0.01 to 5% by weight, and
- optionally one or more pharmaceutically acceptable excipients selected from the additives such as binders, disintegrants, diluents, glidants and lubricants.

5. The pharmaceutical composition prepared according to claim 3, **characterized in that** the pharmaceutical composition comprises, based on the weight of the core tablet,
- ezetimibe or a pharmaceutically acceptable salt thereof in an amount of from about 0.1 to 20% by weight,
- atorvastatin or a pharmaceutically acceptable salt thereof in an amount of from about 1 to 40% by weight,
- a pharmaceutically acceptable surfactant in an amount of from about 0.01 to 5% by weight,
- at least one pharmaceutically acceptable diluent in an amount of more than about 60% by weight, and
- optionally one or more pharmaceutically acceptable excipients selected from the additives such as binders, disintegrants, glidants and lubricants.

6. The pharmaceutical composition prepared according to claim 3, **characterized in that** the pharmaceutical composition comprises calcium salt of atorvastatin as a pharmaceutically acceptable salt of atorvastatin.

7. The pharmaceutical composition prepared according to claim 3, **characterized in that** the pharmaceutical composition comprises sodium lauryl sulfate as surfactant.

8. The pharmaceutical composition prepared according to claim 3, **characterized in that** the pharmaceutical composition comprises lactose as diluent.

9. The pharmaceutical composition prepared according to claim 3, **characterized in that** the pharmaceutical composition comprises croscarmellose sodium as disintegrant.

10. The pharmaceutical composition according to claim 9, **characterized in that** the disintegrant is present in an amount within the range of 0 to 10% by weight, more preferably 1 to 5% by weight.

11. The pharmaceutical composition prepared according to claim 3, **characterized in that** the pharmaceutical composition comprises silicon dioxide as glidant.

12. The pharmaceutical composition according to claim 11, **characterized in that** the glidant is present in an amount up to 1% by weight.

13. The pharmaceutical composition prepared according to claim 3, **characterized in that** the pharmaceutical composition comprises magnesium stearate as lubricant.

14. The pharmaceutical composition according to claim 13, **characterized in that** the lubricant is present in an amount within the range of 0 to 10% by weight, more preferably 0.25 to 5% by weight.

15. The pharmaceutical composition according to any of claims 3-14, **characterized in that** the pharmaceutical composition is in the form of a solid dosage form for oral use, preferably in the form of tablet, more preferably a film tablet.

## Patentansprüche

1. Verfahren zur Zubereitung einer pharmazeutischen Zusammensetzung, welche eine therapeutisch wirksame Menge an Ezetimib oder ein pharmazeutisch zulässiges Salz davon sowie eine therapeutisch wirksame Menge an Atorvastatin oder ein pharmazeutisch zulässiges Salz davon umfasst, **dadurch gekennzeichnet, dass** das Ezetimib oder ein pharmazeutisch zulässiges Salz davon sowie das Atorvastatin oder ein pharmazeutisch zulässiges Salz davon in einer Reihe von Herstellverfahrensschritten vorformuliert werden, umfassend:
a) Die Vorformulierung von Atorvastatin zubereiten, indem das Atorvastatin oder ein pharmazeutisch zulässiges Salz davon, ein pharmazeutisch zulässiges Verdünnungsmittel und optional andere pharmazeutisch zulässige Hilfsstoffe vermischt und anschließend gesiebt werden;
b) Die Vorformulierung von Ezetimib aufbereiten, indem das Ezetimib oder ein pharmazeutisch zulässiges Salz davon in einer Lösung aufgelöst wird, wobei die Lösung ein pharmazeutisch zulässiges Netzmittel umfasst, wobei sich das Netzmittel in einem Lösemittel befindet, das ein Zellulosederivat und/oder ein geeignetes reines Lösemittel oder eine Lösemittelmischung umfasst, um somit eine Granulierungslösung zu erhalten, und anschließend das pharmazeutisch zulässige Verdünnungsmittel mit der besagten Granulierungslösung granulieren und danach das Granulat trocknen und sieben;
c) die in Schritt a) zubereitete Vorformulierung von Atorvastatin, die in Schritt b) zubereitete Vorformulierung von Ezetimib und optional andere pharmazeutisch zulässige Hilfsstoffe vermischen.

2. Verfahren nach Anspruch 1, **dadurch gekennzeichnet, dass** beide Vorformulierungen vermischt werden, um somit eine homogene Tablette zu erhalten, wobei die endgültige Mischung optional mit den anderen pharmazeutisch zulässigen Hilfsstoffen vermischt wird und für die Tablettenpresse vorbereitet wird; oder dass beide Vorformulierungen in die Tablettenpressmaschine separat eingespeist werden, um somit eine geschichtete Tablette zu erhalten.

3. Pharmazeutische Zusammensetzung zubereitet nach Anspruch 1 oder Anspruch 2.

4. Pharmazeutische Zusammensetzung zubereitet nach Anspruch 3, **dadurch gekennzeichnet, dass** die pharmazeutische Zusammensetzung, beruhend auf dem Gewicht der Kerntablette,
- Ezetimib oder ein pharmazeutisch zulässiges Salz davon in einer Menge von ungefähr 0,1 bis 20 Gewichtsprozent,
- Atorvastatin oder ein pharmazeutisch zulässiges Salz davon in einer Menge von ungefähr 1 bis 40 Gewichtsprozent,
- ein pharmazeutisch zulässiges Netzmittel in einer Menge von ungefähr 0,01 bis 5 Gewichtsprozent und
- optional ein oder mehrere pharmazeutisch zulässige Hilfsstoffe umfasst, ausgewählt aus Zusatzmitteln wie zum Beispiel Bindemittel, Sprengmittel, Verdünnungsmittel, Gleitmittel und Schmiermittel.

5. Pharmazeutische Zusammensetzung zubereitet nach Anspruch 3, **dadurch gekennzeichnet, dass** die pharmazeutische Zusammensetzung, beruhend auf dem Gewicht der Kerntablette,
- Ezetimib oder ein pharmazeutisch zulässiges Salz davon in einer Menge von ungefähr 0,1 bis 20 Gewichtsprozent,
- Atorvastatin oder ein pharmazeutisch zulässiges Salz davon in einer Menge von ungefähr 1 bis 40 Gewichtsprozent,
- ein pharmazeutisch zulässiges Netzmittel in einer Menge von ungefähr 0,01 bis 5 Gewichtsprozent,
- mindestens ein pharmazeutisch zulässiges Verdünnungsmittel in einer Menge von mehr als 60 Gewichtsprozent und
- optional ein oder mehrere pharmazeutisch zulässige Hilfsstoffe umfasst, ausgewählt aus Zusatzmitteln wie zum Beispiel Bindemittel, Sprengmittel, Gleitmittel und Schmiermittel.

6. Pharmazeutische Zusammensetzung zubereitet nach Anspruch 3, **dadurch gekennzeichnet, dass** die pharmazeutische Zusammensetzung als ein pharmazeutisch zulässiges Salz von Atorvastatin ein Calcium-Salz von Atorvastatin umfasst.

7. Pharmazeutische Zusammensetzung zubereitet nach Anspruch 3, **dadurch gekennzeichnet, dass** die pharmazeutische Zusammensetzung als Netzmittel Natriumlaurylsulfat umfasst.

8. Pharmazeutische Zusammensetzung zubereitet nach Anspruch 3, **dadurch gekennzeichnet, dass** die pharmazeutische Zusammensetzung als Verdünnungsmittel Laktose umfasst.

9. Pharmazeutische Zusammensetzung zubereitet nach Anspruch 3, **dadurch gekennzeichnet, dass** die pharmazeutische Zusammensetzung als Sprengmittel Croscarmellose-Natrium umfasst.

10. Pharmazeutische Zusammensetzung nach Anspruch 9, **dadurch gekennzeichnet, dass** das Sprengmittel in einer Menge im Bereich von 0 bis 10 Gewichtsprozent, vorzugsweise 1 bis 5 Gewichtsprozent vorhanden ist.

11. Pharmazeutische Zusammensetzung zubereitet nach Anspruch 3, **dadurch gekennzeichnet, dass** die pharmazeutische Zusammensetzung als Gleitmittel Siliciumdioxid umfasst.

12. Pharmazeutische Zusammensetzung nach Anspruch 11, **dadurch gekennzeichnet, dass** das Gleitmittel in einer Menge von bis zu 1 Gewichtsprozent vorhanden ist.

13. Pharmazeutische Zusammensetzung zubereitet nach Anspruch 3, **dadurch gekennzeichnet, dass** die pharmazeutische Zusammensetzung als Schmiermittel Magnesiumstearat umfasst.

14. Pharmazeutische Zusammensetzung nach Anspruch 13, **dadurch gekennzeichnet, dass** das Schmiermittel in einer Menge im Bereich von 0 bis 10 Gewichtsprozent, vorzugsweise 0,25 bis 5 Gewichtsprozent vorhanden ist.

15. Pharmazeutische Zusammensetzung nach einem der Ansprüche 3-14, **dadurch gekennzeichnet, dass** die pharmazeutische Zusammensetzung als eine feste Dosierungsform für den oralen Gebrauch, vorzugsweise als eine Tablettenform, bevorzugt als eine Filmtablette ausgestaltet ist.

## Revendications

1. Un procédé pour la préparation d'une composition pharmaceutique comprenant une quantité thérapeutiquement efficace d'ézétimibe ou d'un sel pharmaceutiquement acceptable de celui-ci et une quantité thérapeutiquement efficace d'atorvastatine ou d'un sel pharmaceutiquement acceptable de celle-ci, **caractérisé en ce que** l'ézétimibe ou un sel pharmaceutiquement acceptable de celui-ci et l'atorvastatine ou un sel pharmaceutiquement acceptable de celle-ci sont préformulés dans une série d'étapes de procédé de fabrication comprenant :
a) la préparation de la préformulation d'atorvastatine, par le mélange et ensuite le tamisage de l'atorvastatine ou d'un sel pharmaceutiquement acceptable de celle-ci, d'un diluant pharmaceutiquement acceptable et éventuellement d'autres excipients pharmaceutiquement acceptables conjointement ;
b) la préparation de la préformulation d'ézétimibe en dissolvant l'ézétimibe ou un sel pharmaceutiquement acceptable de celui-ci dans une solution comprenant un agent tensioactif pharmaceutiquement acceptable, l'agent tensioactif étant dans un solvant comprenant un dérivé de cellulose et/ou un solvant pur convenable ou un mélange de solvants et obtenant ainsi une solution de granulation, et ensuite en granulant un diluant pharmaceutiquement acceptable avec ladite solution de granulation, étape suivie du séchage et du tamisage des granulés ;
c) le mélange de la préformulation d'atorvastatine préparée à l'étape a), de la préformulation d'ézétimibe préparée à l'étape b) et éventuellement d'autres excipients pharmaceutiquement acceptables.

2. Le procédé selon la revendication 1, **caractérisé en ce que** les deux préformulations sont mélangées pour obtenir un comprimé homogène, le mélange final est mélangé éventuellement avec les autres excipients pharmaceutiquement acceptables et préparé pour la presse à comprimés ; ou les deux préformulations sont introduites séparément dans la machine de presse à comprimés pour obtenir un comprimé stratifié.

3. Une composition pharmaceutique préparée selon la revendication 1 ou la revendication 2.

4. La composition pharmaceutique préparée selon la revendication 3, **caractérisée en ce que** la composition pharmaceutique comprend, par rapport au poids du comprimé de base,
- l'ézétimibe ou un sel pharmaceutiquement acceptable de celui-ci en une quantité allant d'environ 0,1 à 20 % en poids,
- l'atorvastatine ou un sel pharmaceutiquement acceptable de celle-ci en une quantité allant d'environ 1 à 40 % en poids,
- un agent tensioactif pharmaceutiquement acceptable en une quantité allant d'environ 0,01 à 5 % en poids, et
- éventuellement un ou plusieurs excipients pharmaceutiquement acceptables choisis parmi les additifs tels que liants, délitants, diluants, agents de glissement et lubrifiants.

5. La composition pharmaceutique préparée selon la revendication 3, **caractérisée en ce que** la composition pharmaceutique comprend, par rapport au poids du comprimé de base,
- l'ézétimibe ou un sel pharmaceutiquement acceptable de celui-ci en une quantité allant d'environ 0,1 à 20 % en poids,
- l'atorvastatine ou un sel pharmaceutiquement acceptable de celle-ci en une quantité allant d'environ 1 à 40 % en poids,
- un agent tensioactif pharmaceutiquement acceptable en une quantité allant d'environ 0,01 à 5 % en poids,
- au moins un diluant pharmaceutiquement acceptable en une quantité de plus de 60 % en poids environ, et
- éventuellement un ou plusieurs excipients pharmaceutiquement acceptables choisis parmi les additifs tels que liants, délitants, agents de glissement et lubrifiants.

6. La composition pharmaceutique préparée selon la revendication 3, **caractérisée en ce que** la composition pharmaceutique comprend un sel de calcium d'atorvastatine sous forme de sel pharmaceutiquement acceptable de l'atorvastatine.

7. La composition pharmaceutique préparée selon la revendication 3, **caractérisée en ce que** la composition pharmaceutique comprend du laurylsulfate de sodium comme agent tensioactif.

8. La composition pharmaceutique préparée selon la revendication 3, **caractérisée en ce que** la composition pharmaceutique comprend du lactose comme diluant.

9. La composition pharmaceutique préparée selon la revendication 3, **caractérisée en ce que** la composition pharmaceutique comprend du sodium de croscarmellose comme délitant.

10. La composition pharmaceutique selon la revendication 9, **caractérisée en ce que** le délitant est présent en une quantité comprise dans la gamme allant de 0 à 10 % en poids, plus préférentiellement de 1 à 5 % en poids.

11. La composition pharmaceutique préparée selon la revendication 3, **caractérisée en ce que** la composition pharmaceutique comprend du dioxyde de silicium comme agent de glissement.

12. La composition pharmaceutique selon la revendication 11, **caractérisée en ce que** l'agent de glissement est présent en une quantité allant jusqu'à 1 % en poids.

13. La composition pharmaceutique préparée selon la revendication 3, **caractérisée en ce que** la composition pharmaceutique comprend du stéarate de magnésium comme lubrifiant.

14. La composition pharmaceutique selon la revendication 13, **caractérisée en ce que** le lubrifiant est présent en une quantité comprise dans la gamme allant de 0 à 10 % en poids, plus préférentiellement de 0,25 à 5 % en poids.

15. La composition pharmaceutique selon l'une quelconque des revendications 3 à 14, **caractérisée en ce que** la composition pharmaceutique est présentée sous la forme d'une forme posologique solide à usage oral, de préférence sous forme de comprimé, et plus préférentiellement sous forme de comprimé pelliculé.
